# EUROPÄISCHE PATENTANMELDUNG

(11) **EP 0 727 425 A1**
(43) Veröffentlichungstag der Anmeldung: **21.08.1996**
(21) Anmeldenummer: 96101746.4
(22) Anmeldetag: 07.02.1996
(51) Int. Cl.: C07D 413/06, C07D 413/14, C07D 263/20, A61K 31/445, A61K 31/495

(54) **Adhäsionsrezeptor-Antagonisten**

(30) Priorität: 15.02.1995 DE 19504954
(71) Anmelder: MERCK PATENT GmbH, D-64293 Darmstadt (DE)
(72) Erfinder: Gante, Joachim, Prof., D-64291 Darmstadt (DE); Juraszyk, Horst, Dr., D-64342 Seeheim (DE); Raddatz, Peter, Dr., D-64342 Seeheim (DE); Wurziger, Hanns, Dr., D-64291 Darmstadt (DE); Bernotat-Danielowski, Sabine, Dr., D-61231 Bad Nauheim (DE); Melzer, Guido, Dr., D-65719 Hofheim/Ts (DE)

(57) **Zusammenfassung**

Verbindungen der Formel I 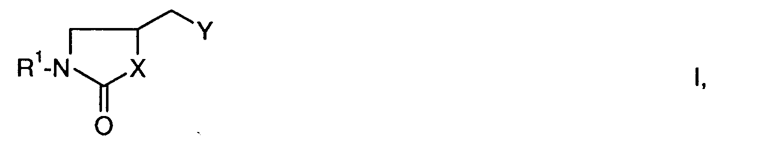 worin R¹ und Y die angegebenen Bedeutungen besitzen, sowie deren physiologisch unbedenklichen Salze, hemmen die Bindung von Fibrinogen an den entsprechenden Rezeptor und können zur Behandlung von Thrombosen, Osteoporosen, Tumorerkrankungen, Apoplexie, Herzinfarkt, Entzündungen, Arteriosklerose und osteolytischen Erkrankungen eingesetzt werden.

## Beschreibung

Die Erfindung betrifft Verbindungen der Formel I 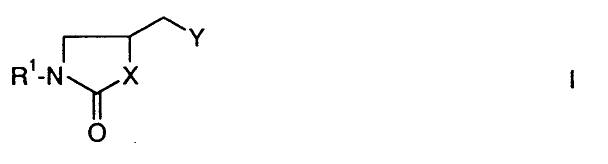 worin
- X: O, S, NH oder NA,
- Y: 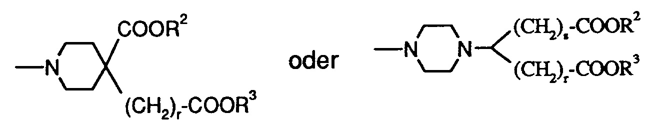
- R¹: 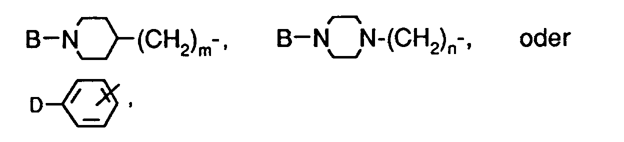
- R² und R³: jeweils unabhängig voneinander H, A oder Benzyl,
- A: Alkyl mit 1 bis 6 C-Atomen,
- B: H, A, Benzyl oder Amidino,
- D: Amidino, Aminomethyl, Aminohydroxyiminomethyl, 5-Oxo-1,2,4-oxadiazolin-3-yl, 5-Methyl-1,2,4-oxadiazolin-3-yl oder Guanidinomethyl,
- m, r und s: jeweils unabhängig voneinander 0, 1, 2, 3 oder 4
und
- n: 2, 3 oder 4,
bedeuten,
sowie ihre physiologisch unbedenklichen Salze.

Ähnliche, jedoch strukturell verschiedene Verbindungen sind aus der EP-A1-0 381 033 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Diese Aufgabe wurde durch die Erfindung gelöst. Es wurde gefunden, daß die Verbindungen der Formel I sowie ihre Solvate und Salze bei guter Verträglichkeit wertvolle pharmakologische Eigenschaften besitzen. Insbesondere hemmen sie die Bindung von Fibrinogen, Fibronectin und des von-Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die Verbindungen beeinflussen somit Zell-Zell- und Zell-Matrix-Wechselwirkungen. Sie verhindern insbesondere die Entstehung von Blutplättchenthromben und können daher zur Behandlung von Thrombosen, Apoplexie, Herzinfarkt, Ischämien, Entzündungen, Arteriosklerose und von akutem Nierenversagen verwendet werden. Ferner haben die Verbindungen einen Effekt auf Tumorzellen, indem sie deren Metastasierung hemmen. Somit können sie auch als Anti-Tumor-Mittel eingesetzt werden.

Es gibt Hinweise, daß Tumorzellen durch Mikrothrombi in die Gefäße gelangen und somit vor der Detektion durch die Zellen des Immunsystems geschützt sind. Ebenso wirken Mikrothrombi unterstützend auf die Bindung der Tumorzellen an die Gefäßwände. Da die Bildung der Mikrothrombi im Zusammenhang mit der Fibrinogen-Bindung zum Fibrinogen-Rezeptor (Glycoprotein IIb/IIIa) steht, gelten Fibrinogen-Bindungs-Inhibitoren ebenfalls als Metastase-Inhibitoren.

Die Verbindungen eignen sich zudem als antimikrobielle Wirkstoffe, die Infektionen, wie sie beispielsweise durch Bakterien, Pilze oder Hefen ausgelöst werden, verhindern können. Die Substanzen können daher vorzugsweise als begleitende antimikrobielle Wirkstoffe gegeben werden, wenn Eingriffe an Organismen vorgenommen werden, bei denen körperfremde Stoffe, wie z.B. Biomaterialien, Implantate, Katheter oder Herzschrittmacher, eingesetzt werden. Sie wirken als Antiseptika. Antimikrobielle Aktivitäten der Verbindungen können z.B. nach der Methode von P. Valentin-Weigand et al., beschrieben in Infection and Immunity, 2851-2855 (1988), nachgewiesen werden.

Die anderen Eigenschaften der Verbindungen können nach Methoden nachgewiesen werden, die in der EP-A1-0 462 960 beschrieben sind. Die Hemmung der Fibrinogenbindung an den Fibrinogenrezeptor kann nach der Methode nachgewiesen werden, die in der EP-A1-0 381 033 angegeben ist. Die thrombozytenaggregationshemmende Wirkung läßt sich in vitro nach der Methode von Born (Nature 4832, 927-929, 1962) nachweisen.

Gegenstand der Erfindung ist ferner ein Verfahren zur Herstellung einer Verbindung der angegebenen Formel I sowie von deren Salzen, dadurch gekennzeichnet, daß man
(a) eine Verbindung der Formel I aus einem ihrer funktionellen Derivate durch Behandeln mit einem solvolysierenden oder hydrogenolysierenden Mittel in Freiheit setzt, oder daß man
(b) eine Verbindung der Formel II 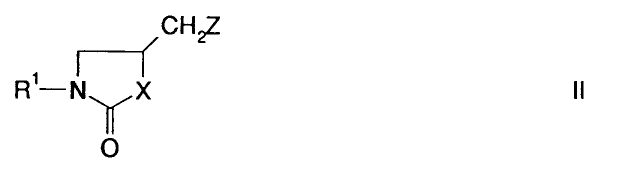 worin
   - Z: Cl, Br, I oder eine reaktionsfähig veresterte OH-Gruppe
   bedeutet, und
   - R¹ und X: die oben angegebenen Bedeutungen haben,
   mit einer Verbindung der Formel III

   H-Y III,

   worin
   - Y: die angegebene Bedeutung besitzt,
   umsetzt, oder daß man
(c) eine Verbindung der Formel IV

   R¹-NH-CH₂-CH(XH)-CH₂-Y IV,

   worin
   - R¹, X und Y: die angegebenen Bedeutungen haben,
   mit einem reaktionsfähigen Derivat der Kohlensäure umsetzt, oder daß man
(d) eine Verbindung der Formel V 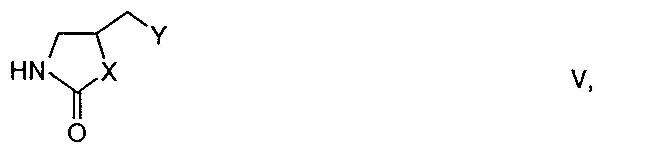 worin
   - X und Y: die angegebenen Bedeutungen haben, mit einer Verbindung der Formel VI 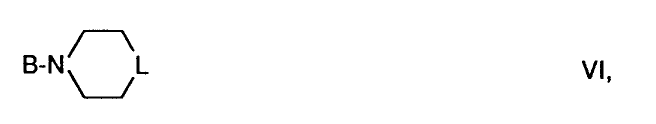
   worin
   - B: die angegebene Bedeutung hat
   und
   - L: 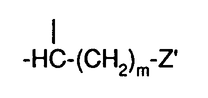 oder 〉N-(CH₂)ₙ-Z',
   wobei
   - Z': Cl, Br, I, OA, OH oder eine reaktionsfähige veresterte OH-Gruppe bzw. leicht nucleophil substituierbare Abgangsgruppe bedeutet,
   umsetzt, oder daß man
(e) (einen) Rest(e) R¹ und/oder Y in (einen) andere(n) Rest(e) R¹ und/oder Y umwandelt, und/oder daß man
(f) eine Verbindung der Formel I durch Behandeln mit einer Säure oder Base in eines ihrer Salze überführt.

Die Verbindungen der Formel I besitzen mindestens ein chirales Zentrum und können daher in mehreren enantiomeren Formen auftreten. Alle diese Formen (z.B. D- und L-Formen) und deren Gemische (z.B. die DL-Formen) sind in der Formel I eingeschlossen.

Vor- und nachstehend haben die Reste bzw. Parameter A, B, L, X, Y, Z, Z', R¹ bis R³, m, n und r die bei den Formeln I bis VI angegebenen Bedeutungen, falls nicht ausdrücklich etwas anderes angegeben ist. Falls mehrere gleich bezeichnete Gruppen im Molekül vorhanden sind, können sie unabhängig voneinander, verschiedene Definitionen annehmen.

In den vorstehenden Formeln hat die Gruppe A 1-6, vorzugsweise 1, 2, 3 oder 4 C-Atome. Im einzelnen bedeutet A vorzugsweise Methyl, Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl.

R¹ ist vorzugsweise ein 4-Piperidyl-, 1-Amidino-4-piperidyl-, 4-Piperidylmethyl-, 4-Piperidylethyl-, 1-Methyl-4-piperidyl-, 1-Amidino-4-piperidylmethyl-, 4-Piperazinylethyl-, 4-Piperazinylpropyl-, 4-Piperazinylbutyl-, 1-Amidino-4-piperazinylethyl- oder ein 1-Amidino-4-piperazinylpropyl-Rest. Besonders bevorzugt ist R¹ jedoch 2-, 3- oder 4-Amidinophenyl, 4-(Aminohydroxyiminomethyl)-phenyl, 2-, 3- oder 4-Aminomethylphenyl, 2-, 3- oder 4-Guanidinomethylphenyl, 4-(5-Oxo-1,2,4-oxadiazolin-3-yl)-phenyl oder 4-(5-Methyl-1,2,4-oxadiazolin-3-yl)-phenyl.

R² und R³ stehen jeweils unabhängig voneinander vorzugsweise für Wasserstoff, Methyl, Ethyl oder Benzyl bedeutet.

B ist bevorzugt Amidino, während D vorzugsweise Amidino oder Aminomethyl bedeutet.

X steht vorzugsweise für Sauerstoff.

Der Parameter m ist vorzugsweise 1, oder 2. Der Parameter n ist vorzugsweise 2 oder 3, und r bedeutet bevorzugt 1, 2 oder 3.

Unter den Verbindungen der Formel I sind diejenigen bevorzugt, in denen mindestens einer der angegebenenReste, Gruppen und/oder Parameter eine der angegebenen bevorzugten Bedeutungen hat. Einige Gruppen von bevorzugten Verbindungen sind diejenigen der Formeln Ia bis Ih, die der Formel I entsprechen, worin jedoch
- in Ia X: Sauerstoff und R¹ 4-Amidinophenyl bedeutet;
- in Ib X: Sauerstoff und R¹ 1-Amidino-4-piperidyl bedeutet;
- in Ic X: Sauerstoff und R¹ 4-(Aminohydroxyiminomethyl)-phenyl oder 4-(5-Methyl-1,2,4-oxadiazolin-3-yl)-phenyl bedeutet;
- in Id X: Sauerstoff und R¹ 4-Piperidylmethyl, -ethyl oder -propyl bedeutet;
- in Ie X: Sauerstoff und R¹ 1-Amidino-4-piperidylmethyl-, ethyl oder - propyl bedeutet;
- in If X: Sauerstoff und R¹ 4-Amidinophenyl oder 4-Aminomethylphenyl bedeutet;
- in Ig X: Sauerstoff und R¹ 1-Piperazinylethyl- oder 1-Amidino-4-piperazinylethyl bedeutet;
- in Ih X: Sauerstoff und R¹ 1-Piperazinylpropyl- oder 1-Amidino-4-piperazinylpropyl bedeutet.

Weiterhin sind bevorzugt Verbindungen der Formeln Ii sowie Iai bis Ihi, die den Formeln I bzw. Ia bis Ih entsprechen, worin jedoch zusätzlich
- Y: 4-Ethoxycarbonyl-4-ethoxycarbonylmethyl-piperidino, 4-[1,2-Di-ethoxycarbonyl-ethyl]-piperazino, 4-[1,2-Dibenzyloxycarbonyl-ethyl]-piperazino oder 4-(1,2-Dicarboxyethyl)-piperazino bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A1-0 381 033, EP-A1-0 462 960) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die an Stelle des H-Atoms einer Hydroxygruppe eine Hydroxyschutzgruppe tragen, z.B. solche, die der Formel I entsprechen, jedoch anstelle einer Gruppe -COOH eine Gruppe -COOR'' tragen, worin R'' eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkyl-gruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe anstelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, Sulfoxide wie Dimethylsulfoxid (DMSO), ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-60° abgespalten weden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können bevorzugt auch durch Reaktion einer Verbindung der Formel II mit einem Amin der Formel III erhalten werden. Dabei bedient man sich zweckmäßig der an sich bekannten Methoden der N-Alkylierung.

Die Fluchtgruppe Z bedeutet vorzugsweise Cl, Br, I, C₁-C₆-Alkylsulfonyloxy wie Methan- oder Ethansulfonyloxy oder C₆-C₁₀-Arylsulfonyloxy wie Benzol-, p-Toluol- oder 1- oder 2-Naphthalinsulfonyloxy.

Die Reaktion gelingt vorzugsweise in Gegenwart einer zusätzlichen Base, z.B. eines Alkali- oder Erdalkalimetall-hydroxids oder carbonats wie Natrium-, Kalium- oder Calciumhydroxid, Natrium-, Kalium- oder Calciumcarbonat, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 200, vorzugsweise zwischen 0 und 120°. Falls die Fluchtgruppe Z von I verschieden ist, empfiehlt sich ein Zusatz eines Iodids wie Kaliumiodid.

Die Ausgangsstoffe der Formel II sind in der Regel neu. Sie können z.B. hergestellt werden durch Reaktion eines substituierten Phenyl-, Piperidin- oder Piperazinderivates der Formel R¹-NH₂ mit einer Verbindung der Formel R⁵CH₂-CHR⁶-CH₂OH (worin R⁵ Z, R⁶ XR⁷, R⁷ eine Schutzgruppe, R⁵ und R⁶ zusammen auch O bedeuten) zu einer Verbindung der Formel R¹-NH-CH₂-CHR⁸-CH₂OH (worin R⁸ XR⁷ oder OH bedeutet), gegebenenfalls Abspaltung der Schutzgruppe R⁷ zu Verbindungen der Formel R¹-NH-CH₂-CH(XH)-CH₂OH, Reaktion mit einem Derivat der Kohlensäure wie Diethylcarbonat zu 3-R¹-5-hydroxymethyl-2-oxazolidinonen und Umwandlung der Hydroxymethylgruppe in eine CH₂Z-Gruppe, z.B. mit SOCl₂, SOBr₂, Methansulfonylchlorid oder p-Toluolsulfonylchlorid. Die Verbindungen der Formel H-Y (III) sind in der Regel bekannt oder in Analogie zu bekannten Verbindungen herstellbar.

Verbindungen der Formel I können ferner erhalten werden durch Reaktion einer Verbindung der Formel IV (oder eines reaktionsfähigen Derivats davon) mit einem reaktiven Derivat der Kohlensäure.

Als Kohlensäurederivate eignen sich insbesondere Dialkylcarbonate wie Diethylcarbonat, ferner auch Chlorameisensäurealkylester wie Ethyl-chlorformiat. Bevorzugt dient das Kohlensäurederivat, das zweckmäßig im Überschuß eingesetzt wird, auch als Lösungs- bzw. Suspensionsmittel. Es kann aber auch eines der angegebenen Lösungsmittel anwesend sein, sofern es bei dieser Umsetzung inert ist. Weiterhin empfiehlt sich der Zusatz einer Base, insbesondere eines Alkalimetallalkoholats wie Kalium-tert.-butylat. Man arbeitet zweckmäßig bei Reaktionstemperaturen zwischen 0 und 150°, vorzugsweise zwischen 70 und 120°.

Die Ausgangsstoffe der Formel IV sind in der Regel neu. Sie sind z.B. erhältlich durch Funktionalisierung der oben genannten Verbindungen der Formel R¹-NH-CH₂-CH(XH)-CH₂OH zu Verbindungen der Formel R¹-NH-CH₂-CH(XH)-CH₂-E und Reaktion mit Verbindungen der Formel H-Y (III).

Zur Herstellung von Verbindungen der Formel I, worin R¹ eine 1-Amidinopiperidinyl- oder -piperazinylgruppe bedeutet, kann man eine entsprechende Piperidin- oder Piperazinverbindung mit einem amidinierenden Mittel behandeln. Als amidinierendes Mittel ist 1-Amidino-3,5-dimethylpyrazol bevorzugt, das insbesondere in Form seines Nitrats eingesetzt wird. Man arbeitet zweckmäßig unter Zusatz einer Base wie Triethylamin oder Ethyl-diisopropylamin in einem inerten Lösungsmittel oder Lösungsmittelgemisch, z.B. Wasser/Dioxan bei Temperaturen zwischen 0 und 120°, vorzugsweise 60 und 120°.

Ferner kann man eine Verbindung der Formel I herstellen, indem man eine Verbindung der Formel V mit einem Piperidin- oder Piperazinderivat der Formel VI umsetzt.

Verbindungen der Formel V können durch Umsetzung einer Verbindung der Formel H₂N-CH₂-CH(XH)-CH₂-Y, worin X und Y die bereits angegebenen Bedeutungen haben, mit einem reaktiven Kohlensäurederivat erhalten werden. Die Reaktionsbedingungen und die geeigneten Kohlensäurederivate entsprechen den zuvor gemachten Angaben.

Die Verbindungen der Formel VI sind in der Regel bekannt oder können nach an sich bekannten Methoden erhalten werden, diejenigen mit B≠H z.B. durch Funktionalisierung entsprechender Piperidin- oder Piperazinderivate der Formel VI (B=H).

Die Umsetzungen können zweckmäßig nach den an sich bekannten Methoden der N-Alkylierung von Aminen, insbesondere cyclischer Amine, wie sie zuvor für die Verbindungen II und III beschrieben wurden, durchgeführt werden.

Weiterhin ist es möglich, in einer Verbindung der Formel I einen oder beide der Reste R¹ und/oder Y in (einen) andere(n) Rest(e) R¹ und/oder Y umzuwandeln.

Beispielsweise kann man eine 4-(5-Oxo-1,2,4-oxadiazolin-3-yl)-phenyl-, eine 4-(5-Methyl-1,2,4-oxadiazolin-3-yl)-phenyl- oder eine 4-(Aminohydroxyiminomethyl)-Gruppe durch Hydrierung, z.B. mit Raney-Nickel, in eine 4-Amidinophenyl-Gruppe umwandeln.

Insbesondere kann man Carboxylgruppen verestern, Estergruppen spalten, Benzylgruppen hydrogenolytisch entfernen oder Aminogruppen mit einem amidinierenden Mittel behandeln. Ferner kann man konventionelle Amino- oder Hydroxyschutzgruppen einführen oder abspalten.

Zur Veresterung kann man eine Säure der Formel I (R³ = H) mit einem Überschuß eines Alkohols der Formel R³-OH (R³ = A oder Benzyl) behandeln, zweckmäßig in Gegenwart einer starken Säure wie Salzsäure oder Schwefelsäure bei Temperaturen zwischen 0 und 100, vorzugsweise 20 und 50°.

Umgekehrt kann ein Ester der Formel I (R³ = A oder Benzyl), in die entsprechende Säure der Formel I (R³ = H) umgewandelt werden, zweckmäßig durch Solvolyse nach einer der oben angegebenen Methoden, z.B. mit NaOH oder KOH in Wasser-Dioxan bei Temperaturen zwischen 0 und 40°, vorzugsweise 10 und 30°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Es ist auch möglich, Carbonsäuren der Formel I (R³ = H) durch Umsetzung mit entsprechenden Basen in ihre Metall- oder Ammoniumsalze umzuwandeln, z.B. ihre Natrium-, Kalium- oder Calciumsalze.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch-aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoyl-phenyl-glycin) gefüllten Säule; als Laufmittel eignet sich z.B. ein Gemisch Hexan/Isopropanol/ Acetonitril, z.B. im Volumenverhältnis 82:15:3.

Natürlich ist es auch möglich, optisch-aktive Verbindungen der Formel I nach den oben beschriebenen Methoden zu erhalten, indem man Ausgangsstoffe (z.B. solche der Formel II) verwendet, die bereits optisch-aktiv sind.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate.

Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Pharmaka, insbesondere aber in Analogie zu den in der EP-A-459 256 beschriebenen Verbindungen verabreicht, vorzugsweise in Dosierungen zwischen etwa 5 mg und 1 g, insbesondere zwischen 50 und 500 mg pro Dosierungseinheit. Die tägliche Dosierung liegt vorzugsweise zwischen etwa 0,1 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, filtriert über einen Ionenaustauscher, trennt ab, extrahiert mit Essigsäureethylester, trocknet über Natriumsulfat, lyophilisiert gegebenenfalls, dampft ein und reinigt durch Chromatographie an Kieselgel und/oder Kristallisation.

### Beispiel 1

Zu einer Lösung von 1,2 g 4-Ethoxycarbonyl-4-ethoxycarbonylmethylpiperidin ("A") in 20 ml DMF gibt man 3,0 g 3-[4-(5-Oxo-1,2,4-oxadiazolin-3-yl)-phenyl]-5-methansulfonyloxy-methyl-oxazolidin-2-on [erhältlich durch Reaktion von 4-(5-Oxo-1,2,4-oxadiazolin-3-yl)-anilin mit 2,3-Epoxypropan-1-ol zu N-[4-(5-Oxo-1,2,4-oxadiazolin-3-yl)-phenyl]-2,3-dihydroxy-propylamin, Umsetzung mit Diethylcarbonat in Gegenwart von K-tert.-butylat zu 3-[4-(5-Oxo-1,2,4-oxadiazolin-3-yl)-phenyl]-5-hydroxymethyl-oxazolidin-2-on und anschließende Veresterung mit Methansulfonylchlorid], gelöst in 10 ml DMF, hinzu und rührt 60 Min. bei Raumtemperatur. Nach Entfernung des Lösungsmittels und üblicher Aufarbeitung erhält man das 3-[4-(5-Oxo-1,2,4-oxadiazolin-3-yl)-phenyl]-5-(4-ethoxycarbonyl-4-ethoxycarbonylmethyl-piperidinomethyl)-oxazolidin-2-on.

Analog erhält man durch Umsetzung von "A"
- mit: 3-(1-Methyl-4-piperidyl)-5-methansulfonyloxy-methyl-oxazolidin-2-on das 3-(1-Methyl-4-piperidyl)-5-(4-ethoxycarbonyl-4-ethoxycarbonyl-methyl-piperidinomethyl)-oxazolidin-2-on;
- mit: 3-(1-N-BOC-Amidino-4-piperidyl)-5-methansulfonyloxy-methyloxazolidin-2-on das 3-(1-N-BOC-Amidino-4-piperidyl)-5-(4-ethoxycarbonyl-4-ethoxycarbonylmethyl-piperidinomethyl)-oxazolidin-2-on;
- mit: 3-(1-Benzyl-4-piperidylmethyl)-5-methansulfonyloxy-methyl-oxazolidin-2-on das 3-(1-Benzyl-4-piperidylmethyl)-5-(4-ethoxycarbonyl-4-ethoxycarbonylmethyl-piperidinomethyl)-oxazolidin-2-on;
- mit: 3-(1-N-BOC-Amidino-4-piperidylmethyl)-5-methansulfonyloxy-methyloxazolidin-2-on das 3-(1-N-BOC-Amidino-4-piperidylmethyl)-5-(4-ethoxycarbonyl-4-ethoxycarbonylmethyl-piperidinomethyl)-oxazolidin-2-on;
- mit: 3-[2-(1-Benzyl-4-piperidyl)-ethyl]-5-methansulfonyloxy-methyl-oxazolidin-2-on das 3-[2-(1-Benzyl-4-piperidyl)-ethyl]-5-(4-ethoxycarbonyl-4-ethoxycarbonylmethyl-piperidinomethyl)-oxazolidin-2-on;
- mit: 3-[2-(1-N-BOC-Amidino-4-piperidyl)-ethyl]-5-methansulfonyloxy-methyl-oxazolidin-2-on das 3-[2-(1-N-BOC-Amidino-4-piperidyl)-ethyl]-5-(4-ethoxycarbonyl-4-ethoxycarbonylmethyl-piperidinomethyl)-oxazolidin-2-on;
- mit: 3-[3-(1-Benzyl-4-piperidyl)-propyl]-5-methansulfonyloxy-methyl-oxazolidin-2-on das 3-[3-(1-Benzyl-4-piperidyl)-propyl]-5-(4-ethoxycarbonyl-4-ethoxycarbonylmethyl-piperidinomethyl)-oxazolidin-2-on;
- mit: 3-[3-(1-N-BOC-Amidino-4-piperidyl)-propyl]-5-methansulfonyloxy-methyl-oxazolidin-2-on das 3-[3-(1-N-BOC-Amidino-4-piperidyl)-propyl]-5-(4-ethoxycarbonyl-4-ethoxycarbonylmethyl-piperidinomethyl)-oxazolidin-2-on;
- mit: 3-[4-(1-Benzyl-4-piperidyl)-butyl]-5-methansulfonyloxy-methyl-oxazolidin-2-on das 3-[4-(1-Benzyl-4-piperidyl)-butyl]-5-(4-ethoxycarbonyl-4-ethoxycarbonylmethyl-piperidinomethyl)-oxazolidin-2-on;
- mit: 3-[4-(1-N-BOC-Amidino-4-piperidyl)-butyl]-5-methansulfonyloxy-methyl-oxazolidin-2-on das 3-[4-(1-N-BOC-Amidino-4-piperidyl)-butyl]-5-(4-ethoxycarbonyl-4-ethoxycarbonylmethyl-piperidinomethyl)-oxazolidin-2-on;
- mit: 3-[2-(1-Benzyl-4-piperazinyl)-ethyl]-5-methansulfonyloxy-methyl-oxazolidin-2-on das 3-[2-(1-Benzyl-4-piperazinyl)-ethyl]-5-(4-ethoxycarbonyl-4-ethoxycarbonylmethyl-piperidinomethyl)-oxazolidin-2-on;
- mit: 3-[2-(1-N-BOC-Amidino-4-piperazinyl)-ethyl]-5-methansulfonyloxy-methyl-oxazolidin-2-on das 3-[2-(1-N-BOC-Amidino-4-piperazinyl)-ethyl]-5-(4-ethoxycarbonyl-4-ethoxycarbonylmethyl-piperidinomethyl)-oxazolidin-2-on;
- mit: 3-[3-(1-Benzyl-4-piperazinyl)-propyl]-5-methansulfonyloxy-methyl-oxazolidin-2-on das 3-[3-(1-Benzyl-4-piperazinyl)-propyl]-5-(4-ethoxycarbonyl-4-ethoxycarbonylmethyl-piperidinomethyl)-oxazolidin-2-on;
- mit: 3-[3-(1-N-BOC-Amidino-4-piperazinyl)-propyl]-5-methansulfonyloxy-methyl-oxazolidin-2-on das 3-[3-(1-N-BOC-Amidino-4-piperazinyl)-propyl]-5-(4-ethoxycarbonyl-4-ethoxycarbonylmethyl-piperidinomethyl)-oxazolidin-2-on;
- mit: 3-[4-(1-Benzyl-4-piperazinyl)-butyl]-5-methansulfonyloxy-methyl-oxazolidin-2-on das 3-[4-(1-Benzyl-4-piperazinyl)-butyl]-5-(4-ethoxycarbonyl-4-ethoxycarbonylmethyl-piperidinomethyl)-oxazolidin-2-on;
- mit: 3-[4-(1-N-BOC-Amidino-4-piperazinyl)-butyl]-5-methansulfonyloxy-methyl-oxazolidin-2-on das 3-[4-(1-N-BOC-Amidino-4-piperazinyl)-butyl]-5-(4-ethoxycarbonyl-4-ethoxycarbonylmethyl-piperidinomethyl)-oxazolidin-2-on;
- mit: 3-(1-Methyl-4-piperidyl)-5-methansulfonyloxy-methyl-oxazolidin-2-on das 3-(1-Methyl-4-piperidyl)-5-(4-ethoxycarbonyl-4-ethoxycarbonylmethyl-piperidinomethyl)-oxazolidin-2-on;
- mit: 3-(1-Isopropyl-4-piperidyl)-5-methansulfonyloxy-methyl-oxazolidin-2-on das 3-(1-Isopropyl-4-piperidyl)-5-(4-ethoxycarbonyl-4-ethoxycarbonylmethyl-piperidinomethyl)-oxazolidin-2-on;
- mit: 3-(1-tert.-Butyl-4-piperidylmethyl)-5-methansulfonyloxy-methyl-oxazolidin-2-on das 3-(1-tert.-Butyl-4-piperidylmethyl)-5-(4-ethoxycarbonyl-4-ethoxycarbonylmethyl-piperidinomethyl)-oxazolidin-2-on;
- mit: 3-(1-Ethyl-4-piperidylmethyl)-5-methansulfonyloxy-methyl-oxazolidin-2-on das 3-(1-Ethyl-4-piperidylmethyl)-5-(4-ethoxycarbonyl-4-ethoxycarbonylmethyl-piperidinomethyl)-oxazolidin-2-on;
- mit: 3-[2-(1-Isopropyl-4-piperidyl)-ethyl]-5-methansulfonyloxy-methyl-oxazolidin-2-on das 3-[2-(1-Isopropyl-4-piperidyl)-ethyl]-5-(4-ethoxycarbonyl-4-ethoxycarbonylmethyl-piperidinomethyl)-oxazolidin-2-on;
- mit: 3-[2-(1-Methyl-4-piperazinyl)-ethyl]-5-methansulfonyloxy-methyl-oxazolidin-2-on das 3-[2-(1-Methyl-4-piperazinyl)-ethyl]-5-(4-ethoxycarbonyl-4-ethoxycarbonylmethyl-piperidinomethyl)-oxazolidin-2-on;
- mit: 3-[3-(1-Ethyl-4-piperazinyl)-propyl]-5-methansulfonyloxy-methyl-oxazolidin-2-on das 3-[3-(1-Ethyl-4-piperazinyl)-propyl]-5-(4-ethoxycarbonyl-4-ethoxycarbonylmethyl-piperidinomethyl)-oxazolidin-2-on;
- mit: 3-[3-(1-Isopropyl-4-piperazinyl)-propyl]-5-methansulfonyloxy-methyl-oxazolidin-2-on das 3-[3-(1-Isopropyl-4-piperazinyl)-propyl]-5-(4-ethoxycarbonyl-4-ethoxycarbonylmethyl-piperidinomethyl)-oxazolidin-2-on;
- mit: 3-[4-(1-Propyl-4-piperazinyl)-butyl]-5-methansulfonyloxy-methyl-oxazolidin-2-on das 3-[4-(1-Propyl-4-piperazinyl)-butyl]-5-(4-ethoxycarbonyl-4-ethoxycarbonylmethyl-piperidinomethyl)-oxazolidin-2-on.

### Beispiel 2

Analog Beispiel 1 erhält man durch Umsetzung von 0,9 g 1-(1,2-Di-ethoxycarbonyl-ethyl)-piperazin ("B") mit einem Äquivalent 3-[4-(5-Oxo-1,2,4-oxadiazolin-3-yl)-phenyl]-5-methansulfonyloxy-methyl-oxazolidin-2-on [erhältlich wie in Bsp. 1 beschrieben] nach Entfernung des Lösungsmittels und üblicher Aufarbeitung das 3-[4-(5-Oxo-1,2,4-oxadiazolin-3-yl)-phenyl]-5-[4-(1,2-di-ethoxycarbonyl-ethyl)-piperazinomethyl]-oxazolidin-2-on.

Analog erhält man durch Umsetzung von "B"
- mit: 3-(1-Methyl-4-piperidyl)-5-methansulfonyloxy-methyl-oxazolidin-2-on das 3-(1-Methyl-4-piperidyl)-5-[4-(1,2-di-ethoxycarbonyl-ethyl)-piperazinomethyl]-oxazolidin-2-on;
- mit: 3-(1-N-BOC-Amidino-4-piperidyl)-5-methansulfonyloxy-methyl-oxazolidin-2-on das 3-(1-N-BOC-Amidino-4-piperidyl)-5-[4-(1,2-di-ethoxycarbonyl-ethyl)-piperazinomethyl]-oxazolidin-2-on;
- mit: 3-(1-Benzyl-4-piperidylmethyl)-5-methansulfonyloxy-methyl-oxazolidin-2-on das 3-(1-Benzyl-4-piperidylmethyl)-5-[4-(1,2-di-ethoxycarbonyl-ethyl)-piperazinomethyl]-oxazolidin-2-on;
- mit: 3-(1-N-BOC-Amidino-4-piperidylmethyl)-5-methansulfonyloxy-methyl-oxazolidin-2-on das 3-(1-N-BOC-Amidino-4-piperidylmethyl)-5-[4-(1,2-di-ethoxycarbonyl-ethyl)-piperazinomethyl]-oxazolidin-2-on;
- mit: 3-[2-(1-Benzyl-4-piperidyl)-ethyl]-5-methansulfonyloxy-methyl-oxazolidin-2-on das 3-[2-(1-Benzyl-4-piperidyl)-ethyl]-5-[4-(1,2-di-ethoxycarbonyl-ethyl)-piperazinomethyl]-oxazolidin-2-on;
- mit: 3-[2-(1-N-BOC-Amidino-4-piperidyl)-ethyl]-5-methansulfonyloxy-methyl-oxazolidin-2-on das 3-[2-(1-N-BOC-Amidino-4-piperidyl)-ethyl]-5-[4-(1,2-di-ethoxycarbonyl-ethyl)-piperazino- methyl]-oxazolidin-2-on;
- mit: 3-[3-(1-Benzyl-4-piperidyl)-propyl]-5-methansulfonyloxy-methyl-oxazolidin-2-on das 3-[3-(1-Benzyl-4-piperidyl)-propyl]-5-[4-(1,2-di-ethoxycarbonyl-ethyl)-piperazinomethyl]-oxazolidin-2-on;
- mit: 3-[3-(1-N-BOC-Amidino-4-piperidyl)-propyl]-5-methansulfonyloxy-methyl-oxazolidin-2-on das 3-[3-(1-N-BOC-Amidino-4-piperidyl)-propyl]-5-[4-(1,2-di-ethoxycarbonyl-ethyl)-piperazino-methy]-oxazolidin-2-on;
- mit: 3-[4-(1-Benzyl-4-piperidyl)-butyl]-5-methansulfonyloxy-methyl-oxazolidin-2-on das 3-[4-(1-Benzyl-4-piperidyl)-butyl]-5-[4-(1,2-di-ethoxycarbonyl-ethyl)-piperazinomethyl]-oxazolidin-2-on;
- mit: 3-[4-(1-N-BOC-Amidino-4-piperidyl)-butyl]-5-methansulfonyloxy-methyl-oxazolidin-2-on das 3-[4-(1-N-BOC-Amidino-4-piperidyl)-butyl]-5-[4-(1,2-di-ethoxycarbonyl-ethyl)-piperazino-methyl]-oxazolidin-2-on;
- mit: 3-[2-(1-Benzyl-4-piperazinyl)-ethyl]-5-methansulfonyloxy-methyl-oxazolidin-2-on das 3-[2-(1-Benzyl-4-piperazinyl)-ethyl]-5-[4-(1,2-di-ethoxycarbonyl-ethyl)-piperazinomethyl]-oxazolidin-2-on;
- mit: 3-[2-(1-N-BOC-Amidino-4-piperazinyl)-ethyl]-5-methansulfonyloxy-methyl-oxazolidin-2-on das 3-[2-(1-N-BOC-Amidino-4-piperazinyl)-ethyl]-5-[4-(1,2-di-ethoxycarbonyl-ethyl)-piperazino-methyl]-oxazolidin-2-on;
- mit: 3-[3-(1-Benzyl-4-piperazinyl)-propyl]-5-methansulfonyloxy-methyl-oxazolidin-2-on das 3-[3-(1-Benzyl-4-piperazinyl)-propyl]-5-[4-(1,2-di-ethoxycarbonyl-ethyl)-piperazinomethyl]-oxazolidin-2-on;
- mit: 3-[3-(1-N-BOC-Amidino-4-piperazinyl)-propyl]-5-methansulfonyloxy-methyl-oxazolidin-2-on das 3-[3-(1-N-BOC-Amidino-4-piperazinyl)-propyl]-5-[4-(1,2-di-ethoxycarbonyl-ethyl)-piperazino-methyl]-oxazolidin-2-on;
- mit: 3-[4-(1-Benzyl-4-piperazinyl)-butyl]-5-methansulfonyloxy-methyl-oxazolidin-2-on das 3-[4-(1-Benzyl-4-piperazinyl)-butyl]-5-[4-(1,2-di-ethoxycarbonyl-ethyl)-piperazinomethyl]-oxazolidin-2-on;
- mit: 3-[4-(1-N-BOC-Amidino-4-piperazinyl)-butyl]-5-methansulfonyloxy-methyl-oxazolidin-2-on das 3-[4-(1-N-BOC-Amidino-4-piperazinyl)-butyl]-5-[4-(1,2-di-ethoxycarbonyl-ethyl)-piperazino- methyl]-oxazolidin-2-on;
- mit: 3-(1-Methyl-4-piperidyl)-5-methansulfonyloxy-methyl-oxazolidin-2-on das 3-(1-Methyl-4-piperidyl)-5-[4-(1,2-di-ethoxycarbonyl-ethyl)-piperazinomethyl]-oxazolidin-2-on;
- mit: 3-(1-Isopropyl-4-piperidyl)-5-methansulfonyloxy-methyl-oxazolidin-2-on das 3-(1-Isopropyl-4-piperidyl)-5-[4-(1,2-di-ethoxycarbonyl-ethyl)-piperazinomethyl]-oxazolidin-2-on;
- mit: 3-(1-tert.-Butyl-4-piperidylmethyl)-5-methansulfonyloxy-methyl-oxazolidin-2-on das 3-(1-tert.-Butyl-4-piperidylmethyl)-5-[4-(1,2-di-ethoxycarbonyl-ethyl)-piperazinomethyl]-oxazolidin-2-on;
- mit: 3-(1-Ethyl-4-piperidylmethyl)-5-methansulfonyloxy-methyl-oxazolidin-2-on das 3-(1-Ethyl-4-piperidylmethyl)-5-[4-(1,2-di-ethoxycarbonyl-ethyl)-piperazinomethyl]-oxazolidin-2-on;
- mit: 3-[2-(1-Isopropyl-4-piperidyl)-ethyl]-5-methansulfonyloxy-methyl-oxazolidin-2-on das 3-[2-(1-Isopropyl-4-piperidyl)-ethyl]-5-[4-(1,2-di-ethoxycarbonyl-ethyl)-piperazinomethyl]-oxazolidin-2-on;
- mit: 3-[2-(1-Methyl-4-piperazinyl)-ethyl]-5-methansulfonyloxy-methyl-oxazolidin-2-on das 3-[2-(1-Methyl-4-piperazinyl)-ethyl]-5-[4-(1,2-di-ethoxycarbonyl-ethyl)-piperazinomethyl]-oxazolidin-2-on;
- mit: 3-[3-(1-Ethyl-4-piperazinyl)-propyl]-5-methansulfonyloxy-methyl-oxazolidin-2-on das 3-[3-(1-Ethyl-4-piperazinyl)-propyl]-5-[4-(1,2-di-ethoxycarbonyl-ethyl)-piperazinomethyl]-oxazolidin-2-on;
- mit: 3-[3-(1-Isopropyl-4-piperazinyl)-propyl]-5-methansulfonyloxy-methyl-oxazolidin-2-on das 3-[3-(1-Isopropyl-4-piperazinyl)-propyl]-5-[4-(1,2-di-ethoxycarbonyl-ethyl)-piperazinomethyl]-oxazolidin-2-on;
- mit: 3-[4-(1-Propyl-4-piperazinyl)-butyl]-5-methansulfonyloxy-methyl-oxazolidin-2-on das 3-[4-(1-Propyl-4-piperazinyl)-butyl]-5-[4-(1,2-di-ethoxycarbonyl-ethyl)-piperazinomethyl]-oxazolidin-2-on.

### Beispiel 3

Analog Beispiel 1 erhält man ausgehend von 1,6 g 1-(1,2-Di-ethoxycarbonyl-ethyl)-piperazin ("B") durch Umsetzung mit einem Äquivalent 3-[4-(5-Methyl-1,2,4-oxadiazol-3-yl)-phenyl]-5-methansulfonyloxy-methyl-oxazolidin-2-on [erhältlich durch Reaktion von 4-(5-Methyl-1,2,4-oxadiazol-3-yl)-anilin mit 2,3-Epoxypropan-1-ol zu N-[4-(5-Methyl-1,2,4-oxadiazol-3-yl)-phenyl]-2,3-dihydroxy-propyl-amin, Umsetzung mit Diethylcarbonat in Gegenwart von K-tert.-butylat zu 3-[4-(5-Methyl-1,2,4-oxadiazol-3-yl)-phenyl]-5-hydroxymethyl-oxazolidin-2-on und anschließende Veresterung mit Methansulfonylchlorid] nach üblicher Aufarbeitung das 3-[4-(5-Methyl-1,2,4-oxadiazol-3-yl)-phenyl]-5-[4-(1,2-di-benzyloxycarbonyl-ethyl)-piperazinomethyl)-oxazolidin-2-on, F. 119°.

### Beispiel 4

1,3 g 3-(4-Cyan-phenyl)-5-[4-(1,2-di-ethoxycarbonyl-ethyl)-piperazinomethyl)-oxazolidin-2-on [erhält-lich durch Umsetzung von 4-Cyan-anilin mit 2,3-Epoxypropan-1-ol zu N-[4-Cyan-phenyl]-2,3-dihydro-xy-propyl-amin, Reaktion mit Diethylcarbonat in Gegenwart von K-tert.-butylat zu 3-(4-Cyan-phenyl)-5-hydroxymethyl-oxazolidin-2-on, anschließende Veresterung mit Methansulfonylchlorid und Umsetzung mit "B"] sowie 1,1 g Hydroxylamin-Hydrochlorid werden in 125 ml Ethanol in Gegenwart von 2,24 g Natriumethanolat 2 Stunden gekocht. Anschließend wird die Reaktionsmischung filtriert und im Vakuum eingeengt. Man löst den Rückstand in Wasser, stellt die Lösung mit 2 n Salzsäure auf pH 3 ein und saugt den entstehenden Niederschlag ab. Nach Umkristallisieren aus Wasser/Eisessig erhält man das 3-[4-Amino-(hydroxyimino)methyl-phenyl]-5-[4-(1,2-di-ethoxycarbonyl-ethyl)-piperazinomethyl]-oxazolidin-2-on, F. 186°.

### Beispiel 5

Analog Beispiel 1 erhält man ausgehend von 1-(1,2-Di-benzyloxycarbonyl-ethyl)-piperazin ("C") durch Umsetzung mit 3-(4-N-BOC-Aminomethyl-phenyl)-5(R)-methansulfonyloxy-methyl-oxazolidin-2-on [erhältlich durch Reaktion von 4-N-BOC-Aminomethyl-anilin mit 2,3-Epoxypropan-1-ol zu N-(4-N-BOC-Aminomethyl-phenyl)-2,3-dihydroxy-propyl-amin, Umsetzung mit Diethylcarbonat in Gegenwart von K-tert.-butylat zu 3-(4-N-BOC-Aminomethyl-phenyl)-5-hydroxymethyl-oxazolidin-2-on und anschließende Veresterung mit Methansulfonylchlorid] nach üblicher Aufarbeitung das 3-(4-N-BOC-Aminomethyl-phenyl)-5(R)-[4-(1,2-di-benzyloxycarbonyl-ethyl)-piperazinomethyl]-oxazolidin-2-on.

Analog erhält man durch Umsetzung von "C"
- mit: 3-(1-N-BOC-Amidino-4-piperidyl)-5-methansulfonyloxy-methyl-oxazolidin-2-on das 3-(1-N-BOC-Amidino-4-piperidyl)-5-[4-(1,2-di-benzyloxycarbonyl-ethyl)-piperazinomethyl]-oxazolidin-2-on;
- mit: 3-(1-Benzyl-4-piperidylmethyl)-5-methansulfonyloxy-methyl-oxazolidin-2-on das 3-(1-Benzyl-4-piperidylmethyl)-5-[4-(1,2-di-benzyloxycarbonyl-ethyl)-piperazinomethyl]-oxazolidin-2-on;
- mit: 3-(1-N-BOC-Amidino-4-piperidylmethyl)-5-methansulfonyloxy-methyl-oxazolidin-2-on das 3-(1-N-BOC-Amidino-4-piperidylmethyl)-5-[4-(1,2-di-benzyloxycarbonyl-ethyl)-piperazino-methyl]-oxazolidin-2-on;
- mit: 3-[2-(1-N-BOC-Amidino-4-piperazinyl)-ethyl]-5-methansulfonyloxy-methyl-oxazolidin-2-on das 3-[2-(1-N-BOC-Amidino-4-piperazinyl)-ethyl]-5-[4-(1,2-di-benzyloxycarbonyl-ethyl)-piperazino-methyl]-oxazolidin-2-on;
- mit: 3-[3-(1-Benzyl-4-piperazinyl)-propyl]-5-methansulfonyloxy-methyl-oxazolidin-2-on das 3-[3-(1-Benzyl-4-piperazinyl)-propyl]-5-[4-(1,2-di-benzyloxycarbonyl-ethyl)-piperazinomethyl]-oxazolidin-2-on.

### Beispiel 6

0,9 g 3-(1-N-BOC-Amidino-4-piperidyl)-5-(4-ethoxycarbonyl-4-ethoxycarbonylmethyl-piperidino-methyl)-oxazolidin-2-on [erhältlich nach Bsp. 1] werden in 40 ml 2 N HCl-Lösung auf Dioxanbasis suspendiert und 3 Stunden bei Raumtemperatur gerührt. Nach Entfernung des Lösungsmittels und üblicher Aufarbeitung erhält man 3-(1-Amidino-4-piperidyl)-5-(4-ethoxycarbonyl-4-ethoxycarbonyl-methyl-piperidino-methyl)-oxazolidin-2-on, Hydrochlorid.

Analog erhält man nach Entfernung der BOC-Schutzgruppe der Produkte aus Beispiel 1
3-(1-Amidino-4-piperidylmethyl)-5-(4-ethoxycarbonyl-4-ethoxycarbonylmethyl-piperidinomethyl)-oxazolidin-2-on, Hydrochlorid;
3-[2-(1-Amidino-4-piperidyl)-ethyl]-5-(4-ethoxycarbonyl-4-ethoxycarbonylmethyl-piperidinomethyl)-oxazolidin-2-on, Hydrochlorid;
3-[3-(1-Amidino-4-piperidyl)-propyl]-5-(4-ethoxycarbonyl-4-ethoxycarbonylmethyl-piperidinomethyl)-oxazolidin-2-on, Hydrochlorid;
3-[4-(1-Amidino-4-piperidyl)-butyl]-5-(4-ethoxycarbonyl-4-ethoxycarbonylmethyl-piperidinomethyl)-oxazolidin-2-on, Hydrochlorid;
3-[2-(1-Amidino-4-piperazinyl)-ethyl]-5-(4-ethoxycarbonyl-4-ethoxycarbonylmethyl-piperidinomethyl)-oxazolidin-2-on, Hydrochlorid;
3-[3-(1-Amidino-4-piperazinyl)-propyl]-5-(4-ethoxycarbonyl-4-ethoxycarbonylmethyl-piperidinomethyl)-oxazolidin-2-on, Hydrochlorid;
3-[4-(1-Amidino-4-piperazinyl)-butyl]-5-(4-ethoxycarbonyl-4-ethoxycarbonylmethyl-piperidinomethyl)-oxazolidin-2-on, Hydrochlorid.

### Beispiel 7

Analog Beispiel 6 erhält man ausgehend von den Verbindungen aus Beispiel 2 durch Abspaltung der BOC-Schutzgruppen die folgenden Verbindungen in Form ihrer Hydrochloride:
3-(1-Amidino-4-piperidyl)-5-[4-(1,2-di-ethoxycarbonyl-ethyl)-piperazinomethyl]-oxazolidin-2-on;
3-(1-Amidino-4-piperidylmethyl)-5-[4-(1,2-di-ethoxycarbonyl-ethyl)-piperazinomethyl]-oxazolidin-2-on;
3-[2-(1-Amidino-4-piperidyl)-ethyl]-5-[4-(1,2-di-ethoxycarbonyl-ethyl)-piperazino-methyl]-oxazolidin-2-on;
3-[3-(1-Amidino-4-piperidyl)-propyl]-5-[4-(1,2-di-ethoxycarbonyl-ethyl)-piperazino-methyl]-oxazolidin-2-on;
3-[4-(1-Amidino-4-piperidyl)-butyl]-5-[4-(1,2-di-ethoxycarbonyl-ethyl)-piperazino-methyl]-oxazolidin-2-on;
3-[2-(1-Amidino-4-piperazinyl)-ethyl]-5-[4-(1,2-di-ethoxycarbonyl-ethyl)-piperazino-methyl]-oxazolidin-2-on;
3-[3-(1-Amidino-4-piperazinyl)-propyl]-5-[4-(1,2-di-ethoxycarbonyl-ethyl)-piperazino-methyl]-oxazolidin-2-on;
3-[4-(1-N-BOC-Amidino-4-piperazinyl)-butyl]-5-[4-(1,2-di-ethoxycarbonyl-ethyl)-piperazino-methyl]-oxazolidin-2-on.

Analog Beispiel 6 erhält man ausgehend von den Verbindungen aus Beispiel 5 durch Abspaltung der BOC-Schutzgruppen die folgenden Verbindungen:
3-(4-Aminomethyl-phenyl)-5(R)-[4-(1,2-di-benzyloxycarbonyl-ethyl)-piperazinomethyl]-oxazolidin-2-on, Trihydrochlorid-Hydrat, F. 187°;
3-(1-Amidino-4-piperidyl)-5-[4-(1,2-di-benzyloxycarbonyl-ethyl)-piperazinomethyl]-oxazolidin-2-on;
3-(1-Amidino-4-piperidylmethyl)-5-[4-(1,2-di-benzyloxycarbonyl-ethyl)-piperazino-methyl]-oxazolidin-2-on;
3-[2-(1-Amidino-4-piperazinyl)-ethyl]-5-[4-(1,2-di-benzyloxycarbonyl-ethyl)-piperazino-methyl]-oxazolidin-2-on.

### Beispiel 8

1,1 g 3-(4-Chlormethyl-phenyl)-5(R)-[4-(1,2-di-benzyloxycarbonyl-ethyl)-piperazinomethyl]-oxazolidin-2-on [erhältlich durch Reaktion von 4-Chlormethyl-anilin mit 2,3-Epoxypropan-1-ol zu N-(4-Chlormethyl-phenyl)-2,3-dihydroxy-propyl-amin, Umsetzung mit Diethylcarbonat in Gegenwart von K-tert.-butylat zu 3-(4-Chlormethyl-phenyl)-5-hydroxymethyl-oxazolidin-2-on, anschließende Veresterung mit Methansulfonylchlorid und Umsetzung mit 1-(1,2-Di-benzyloxycarbonyl-ethyl)-piperazin], gelöst in 30 ml DMF, werden bei Raumtemperatur mit 0,9 g frisch hergestelltem Guanidin, gelöst in 10 ml DMF versetzt und zwei Stunden gerührt. Nach Entfernung des Lösungsmittels und üblicher Aufarbeitung erhält man das 3-(4-Guanidinylmethyl-phenyl)-5(R)-[4-(1,2-di-benzyloxycarbonyl-ethyl)-piperazinomethyl]-oxazolidin-2-on.

### Beispiel 9

1,1 g 3-(4-Aminomethyl-phenyl)-5(R)-[4-(1,2-di-benzyloxycarbonyl-ethyl)-piperazinomethyl]-oxazo-lidin-2-on (Hydrochorid-Trihydrat, F. 187°) werden in 30 ml Toluol gelöst und bei Raumtemperatur eine Stunde mit Wasserstoffgas (p = 1 atm) unter katalytischer Wirkung von 150 mg Pd-C (Pd-Gehalt 1 %) behandelt. Anschließend filtriert man die Reaktionsmischung und erhält nach üblicher Aufarbeitung 3-(4-Aminomethyl-phenyl)-5(R)-[4-(1,2-di-carboxy-ethyl)-piperazinomethyl]-oxazolidin-2-on, Trihydrochlorid-Hydrat, F. 200°.

Analog erhält man durch Abspaltung der Benzylgruppen
- aus: 3-(4-Guanidinylmethyl-phenyl)-5(R)-[4-(1,2-di-benzyloxycarbonyl-ethyl)-piperazino-methyl]-oxazolidin-2-on das 3-(4-Guanidinylmethyl-phenyl)-5(R)-[4-(1,2-di-carboxy-ethyl)-piperazino-methyl]-oxazolidin-2-on, F. 262°.

### Beispiel 10

1,3 g 3-[4-(5-Oxo-1,2,4-oxadiazolin-3-yl)-phenyl]-5-(4-ethoxycarbonyl-4-ethoxycarbonyl-methyl-piperidinomethyl)-oxazolidin-2-on [erhältlich nach Bsp. 1] werden in 50 ml Methanol gelöst und an Raney-Nickel hydriert. Anschließend wird die Reaktionsmischung filtriert und das Filtrat im Vakuum eingeengt. Das erhaltene Produkt wird mit 20 ml Essigsäureethylester in der Wärme behandelt und nach Abkühlen abgesaugt. Man erhält 3-(4-Amidino-phenyl)-5-(4-ethoxycarbonyl-4-ethoxycarbonyl-methyl-piperidinomethyl)-oxazolidin-2-on, Hydroiodid, F. 123°.

### Beispiel 11

Analog Beispiel 9 erhält man durch reduktive Spaltung der 5-Oxo-1,2,4-oxadiazolin-Gruppe ausgehend von 3-[4-(5-Oxo-1,2,4-oxadiazolin-3-yl)-phenyl]-5-[4-(1,2-di-ethoxycarbonyl-ethyl)-piperazinomethyl)-oxazolidin-2-on nach üblicher Aufarbeitung das 3-(4-Amidino-phenyl)-5-[4-(1,2-di-ethoxycarbonyl-ethyl)-piperazinomethyl]-oxazolidin-2-on, Acetat, F. 179°.

### Beispiel 12

Zu einer Lösung von 0,3 g 3-(4-Amidino-phenyl)-5-(4-ethoxycarbonyl-4-ethoxycarbonyl-methyl-piperidinomethyl)-oxazolidin-2-on in 20 ml THF fügt man 20 ml 20%ige NaOH-Lösung hinzu und rührt 24 Std. bei Raumtemperatur. Man erhält nach Entfernung des Lösungsmittels und Gefriertrocknung das 3-(4-Amidino-phenyl)-5-(4-carboxy-4-carboxy-methyl-piperidinomethyl)-oxazolidin-2-on-Na-Salz, F. > 330°.

Analog erhält man
- aus: 3-(4-Amidino-phenyl)-5-[4-(1,2-di-ethoxycarbonyl-ethyl)-piperazinomethyl]-oxazolidin-2-on das 3-(4-Amidino-phenyl)-5-[4-(1,2-di-carboxy-ethyl)-piperazinomethyl]-oxazolidin-2-on-Na-Salz, dihydrat, F. > 300°.

### Beispiel 13

0,8 g 3-(4-Amidino-phenyl)-5-(4-ethoxycarbonyl-4-ethoxycarbonyl-methyl-piperidinomethyl)-oxazolidin-2-on [erhältlich nach Bsp. 9] werden in 60 ml Methanol suspendiert, mit 10 ml 2 N NaOH-Lösung ver-setzt und 4 Stunden bei Raumtemperatur gerührt. Nach Entfernung des Lösungsmittels wird der Rück-stand in Wasser aufgenommen, der pH-Wert durch Zugabe von verdünnter HCl auf 3 eingestellt und die Reaktionsmischung über einen Ionenaustauscher filtriert. Nach Extraktion mit Ethylacetat wird das Filtrat wird über MgSO₄ getrocknet. Nach Entfernung des Lösungsmittels und anschließende Gefriertrocknung erhält man das 3-(4-Amidino-phenyl)-5-(4-carboxycarbonyl-4-carboxy-methyl-piperidinomethyl)-oxazolidin-2-on.

Analog erhält man durch Verseifung:
- von: 3-(4-Amidino-phenyl)-5-[4-(1,2-di-ethoxycarbonyl-ethyl)-piperazinomethyl]-oxazolidin-2-on das 3-(4-Amidino-phenyl)-5-[4-(1,2-di-carboxy-ethyl)-piperazinomethyl]-oxazolidin-2-on, Acetat, F. 145°;
- von: 3-(1-Methyl-4-piperidyl)-5-(4-ethoxycarbonyl-4-ethoxycarbonylmethyl-piperidinomethyl)-oxazolidin-2-on das 3-(1-Methyl-4-piperidyl)-5-(4-carboxy-4-carboxymethyl-piperidinomethyl)-oxazolidin-2-on;
- von: 3-(1-Benzyl-4-piperidylmethyl)-5-(4-ethoxycarbonyl-4-ethoxycarbonylmethyl-piperidinomethyl)-oxazolidin-2-on das 3-(1-Benzyl-4-piperidylmethyl)-5-(4-carboxy-4-carboxymethyl-piperidinomethyl)-oxazolidin-2-on;
- von: 3-[2-(1-Benzyl-4-piperidyl)-ethyl]-5-(4-ethoxycarbonyl-4-ethoxycarbonylmethyl-piperidinomethyl)-oxazolidin-2-on das 3-[2-(1-Benzyl-4-piperidyl)-ethyl]-5-(4-carboxy-4-carboxymethyl-piperidinomethyl)-oxazolidin-2-on;
- von: 3-[3-(1-Benzyl-4-piperidyl)-propyl]-5-(4-ethoxycarbonyl-4-ethoxycarbonylmethyl-piperidinomethyl)-oxazolidin-2-on das 3-[3-(1-Benzyl-4-piperidyl)-propyl]-5-(4-carboxy-4-carboxymethyl-piperidinomethyl)-oxazolidin-2-on;
- von: 3-[4-(1-Benzyl-4-piperidyl)-buty]-5-(4-ethoxycarbonyl-4-ethoxycarbonylmethyl-piperidinomethyl)-oxazolidin-2-on das 3-[4-(1-Benzyl-4-piperidyl)-butyl]-5-(4-carboxy-4-carboxymethyl-piperidinomethyl)-oxazolidin-2-on;
- von: 3-[2-(1-Benzyl-4-piperazinyl)-ethyl]-5-(4-ethoxycarbonyl-4-ethoxycarbonylmethyl-piperidinomethyl)-oxazolidin-2-on das 3-[2-(1-Benzyl-4-piperazinyl)-ethyl]-5-(4-carboxy-4-carboxymethyl-piperidinomethyl)-oxazolidin-2-on;
- von: 3-[3-(1-Benzyl-4-piperazinyl)-propyl]-5-(4-ethoxycarbonyl-4-ethoxycarbonylmethyl-piperidinomethyl)-oxazolidin-2-on das 3-[3-(1-Benzyl-4-piperazinyl)-propyl]-5-(4-carboxy-4-carboxymethyl-piperidinomethyl)-oxazolidin-2-on;
- von: 3-[4-(1-Benzyl-4-piperazinyl)-butyl]-5-(4-ethoxycarbonyl-4-ethoxycarbonylmethyl-piperidinomethyl)-oxazolidin-2-on das 3-[4-(1-Benzyl-4-piperazinyl)-butyl]-5-(4-carboxy-4-carboxymethyl-piperidinomethyl)-oxazolidin-2-on;
- von: 3-(1-Methyl-4-piperidyl)-5-(4-ethoxycarbonyl-4-ethoxycarbonylmethyl-piperidinomethyl)-oxazolidin-2-on das 3-(1-Methyl-4-piperidyl)-5-(4-carboxy-4-carboxymethyl-piperidinomethyl)-oxazolidin-2-on;
- von: 3-(1-Isopropyl-4-piperidyl)-5-(4-ethoxycarbonyl-4-ethoxycarbonylmethyl-piperidinomethyl)-oxazolidin-2-on das 3-(1-Isopropyl-4-piperidyl)-5-(4-carboxy-4-carboxymethyl-piperidinomethyl)-oxazolidin-2-on;
- von: 3-(1-tert.-Butyl-4-piperidylmethyl)-5-(4-ethoxycarbonyl-4-ethoxycarbonylmethyl-piperidinomethyl)-oxazolidin-2-on das 3-(1-tert.-Butyl-4-piperidylmethyl)-5-(4-carboxy-4-carboxymethyl-piperidinomethyl)-oxazolidin-2-on;
- von: 3-(1-Ethyl-4-piperidylmethyl)-5-(4-ethoxycarbonyl-4-ethoxycarbonylmethyl-piperidinomethyl)-oxazolidin-2-on das 3-(1-Ethyl-4-piperidylmethyl)-5-(4-carboxy-4-carboxymethyl-piperidinomethyl)-oxazolidin-2-on;
- von: 3-[2-(1-Isopropyl-4-piperidyl)-ethyl]-5-(4-ethoxycarbonyl-4-ethoxycarbonylmethyl-piperidinomethyl)-oxazolidin-2-on das 3-[2-(1-Isopropyl-4-piperidyl)-ethyl]-5-(4-carboxy-4-carboxymethyl-piperidinomethyl)-oxazolidin-2-on;
- von: 3-[2-(1-Methyl-4-piperazinyl)-ethyl]-5-(4-ethoxycarbonyl-4-ethoxycarbonylmethyl-piperidinomethyl)-oxazolidin-2-on das 3-[2-(1-Methyl-4-piperazinyl)-ethyl]-5-(4-carboxy-4-carboxymethyl-piperidinomethyl)-oxazolidin-2-on;
- von: 3-[3-(1-Ethyl-4-piperazinyl)-propyl]-5-(4-ethoxycarbonyl-4-ethoxycarbonylmethyl-piperidinomethyl)-oxazolidin-2-on das 3-[3-(1-Ethyl-4-piperazinyl)-propyl]-5-(4-carboxy-4-carboxymethyl-piperidinomethyl)-oxazolidin-2-on;
- von: 3-[3-(1-Isopropyl-4-piperazinyl)-propyl]-5-(4-ethoxycarbonyl-4-ethoxycarbonylmethyl-piperidinomethyl)-oxazolidin-2-on das 3-[3-(1-Isopropyl-4-piperazinyl)-propyl]-5-(4-carboxy-4-carboxymethyl-piperidinomethyl)-oxazolidin-2-on;
- von: 3-[4-(1-Propyl-4-piperazinyl)-butyl]-5-(4-ethoxycarbonyl-4-ethoxycarbonylmethyl-piperidinomethyl)-oxazolidin-2-on das 3-[4-(1-Propyl-4-piperazinyl)-butyl]-5-(4-carboxy-4-carboxymethyl-piperidinomethyl)-oxazolidin-2-on.

### Beispiel 14

Analog Beispiel 1 erhält man durch Umsetzung von 1,3 g 1-(1,2-Di-ethoxycarbonyl-ethyl)-piperazin ("B") mit einem Äquivalent 3-[4-(5-Methyl-1,2,4-oxadiazolin-3-yl)-phenyl]-5-methansulfonyloxymethyl-oxazolidin-2-on [erhältlich durch Reaktion von 4-Cyan-anilin mit 2,3-Epoxypropan-1-ol zu N-[4-Cyan-phenyl]-2,3-dihydroxy-propyl-amin, Umsetzung mit Diethylcarbonat in Gegenwart von K-tert.-butylat zu 3-(4-Cyan-phenyl)-5-hydroxymethyl-oxazolidin-2-on, Überführung durch Reaktion des Nitrils mit Hydroxylamin und danach mit Essigsäurechlorid zum 3-[4-(5-Methyl-1,2,4-oxadiazolin-3-yl)-phenyl]-5-hydroxymethyl-oxazolidin-2-on und anschließende Veresterung mit Methansulfonylchlorid] nach Entfernung des Lösungsmittels und üblicher Aufarbeitung das 3-[4-(5-Methyl-1,2,4-oxadiazolin-3-yl)-phenyl]-5-[4-(1,2-di-ethoxycarbonyl-ethyl)-piperazinomethyl]-oxazolidin-2-on, F. 126-127°.

Analog erhält man durch Umsetzung von "B"
mit 3-[4-(5-Phenyl-1,2,4-oxadiazolin-3-yl)-phenyl]-5-methansulfonyloxy-methyl-oxazolidin-2-on das
3-[4-(5-Phenyl-1,2,4-oxadiazolin-3-yl)-phenyl]-5-[4-(1,2-di-ethoxycarbonyl-ethyl)-piperazinomethyl]-oxazolidin-2-on.

### Beispiel 15

1,3 g 3-[4-(5-Phenyl-1,2,4-oxadiazolin-3-yl)-phenyl]-5-[4-(1,2-di-ethoxycarbonyl-ethyl)-piperazinomethyl]-oxazolidin-2-on [erhältlich nach Bsp. 14] werden in 50 ml Methanol gelöst und an Raney-Nickel hydriert. Anschließend wird die Reaktionsmischung filtriert und das Filtrat im Vakuum eingeengt. Das erhaltene Produkt wird mit 20 ml Essigsäureethylester in der Wärme behandelt und nach Abkühlen abgesaugt und wie üblich aufgearbeitet. Man erhält das 3-[4-(N-Benzoyl-amidino)-phenyl]-5-[4-(1,2-di-ethoxycarbonyl-ethyl)-piperazinomethyl]-oxazolidin-2-on F. 136°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat werden in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 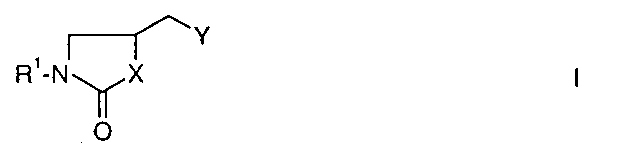 worin
X O, S, NH oder NA,
Y 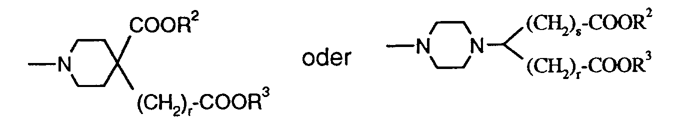
R¹ 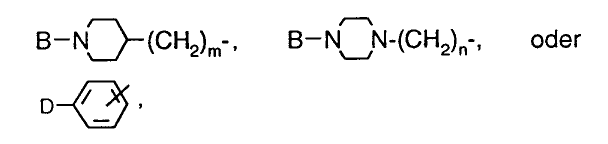
R² und R³ jeweils unabhängig voneinander H, A oder Benzyl,
A Alkyl mit 1 bis 6 C-Atomen,
B H, A, Benzyl oder Amidino,
D Amidino, Aminomethyl, Aminohydroxyiminomethyl, 5-Oxo-1,2,4-oxadiazolin-3-yl, 5-Methyl-1,2,4-oxadiazolin-3-yl oder Guanidinomethyl,
m, r und s jeweils unabhängig voneinander 0, 1, 2, 3 oder 4
und
n 2, 3 oder 4,
bedeuten,
sowie ihre physiologisch unbedenklichen Salze.

2. Enantiomere oder Diastereomere der Verbindungen der Formel I gemäß Anspruch 1

3. Verbindungen der Formel I gemäß Anspruch 1, worin die freien Amino- oder Amidino-gruppen teilweise oder vollständig durch konventionelle Amino-Schutzgruppen geschützt sind.

4. (a) 3-(4-Amidinophenyl)-5-(4-ethoxycarbonyl-4-ethoxycarbonylmethyl-piperidinomethyl)-oxazolidin-2-on;
(b) 3-(4-Amidinophenyl)-5-[4-(1,2-di-ethoxycarbonyl-ethyl)-piperazinomethyl]-oxazolidin-2-on;
(c) 3-(4-Aminomethylphenyl)-5-[4-(1,2-di-benzyloxycarbonyl-ethyl)-piperazinomethyl]-oxazolidin-2-on;
(d) 3-(4-Aminomethylphenyl)-5-[4-(1,2-dicarboxy-ethyl)-piperazinomethyl]-oxazolidin-2-on;
(e) 3-(4-Amidinophenyl)-5-[4-(1,2-dicarboxy-ethyl)-piperazinomethyl]-oxazolidin-2-on;
(f) 3-(4-Guanidinomethylphenyl)-5-[4-(1,2-dicarboxy-ethyl)-piperazinomethyl]-oxazolidin-2-on,
sowie deren physiologisch unbedenklichen Salze, insbesondere deren Alkalimetallsalze.

5. Verfahren zur Herstellung von Verbindungen der Formel I gemäß Anspruch 1, dadurch gekennzeichnet, daß man
(a) eine Verbindung der Formel I aus einem ihrer funktionellen Derivate durch Behandeln mit einem solvolysierenden oder hydrogenolysierenden Mittel in Freiheit setzt, oder daß man
(b) eine Verbindung der Formel II 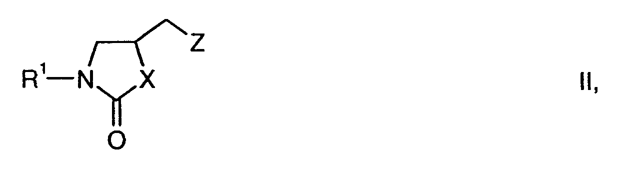 worin
R¹ und X die in Anspruch 1 angegebenen Bedeutungen besitzen
und
Z Cl, Br, I, OH oder eine reaktionsfähig veresterte OH-Gruppe bedeutet,
mit einer Verbindung der Formel III
H-Y III,
worin
Y die angegebene Bedeutung besitzt,
umsetzt, oder daß man
(c) eine Verbindung der Formel IV
R¹-NH-CH₂-CH(XH)-CH₂-Y IV,
worin
R¹, X und Y die angegebenen Bedeutungen haben,
mit einen reaktionsfähigen Derivat der Kohlensäure umsetzt, oder daß man
(d) eine Verbindung der Formel V 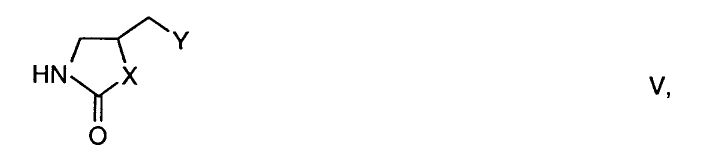 worin
X und Y die angegebenen Bedeutungen haben, mit einer Verbindung der Formel VI 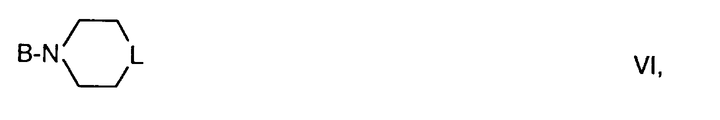
worin
B die angegebene Bedeutung hat
und
L 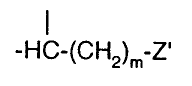 oder 〉N-(CH₂)ₙ-Z',
wobei
Z' Cl, Br, I, OA, OH oder eine reaktionsfähige veresterte OH-Gruppe bzw. leicht nucleophil substituierbare Abgangsgruppe bedeutet,
umsetzt, oder daß man
(e) (einen) Rest(e) R¹ und/oder Y in (einen) andere(n) Rest(e) R¹ und/oder Y umwandelt, und/oder daß man
(f) eine Verbindung der Formel I durch Behandeln mit einer Säure oder Base in eines ihrer Salze überführt.

6. Verfahren zur Herstellung einer pharmazeutischen Zubereitung, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Darreichungsform bringt.

7. Pharmazeutische Zubereitung, gekennzeichnet durch einen Gehalt an mindestens einer Verbindung der Formel I, gemäß Anspruch 1, und/oder einem ihrer physiologisch unbedenklichen Salze.

8. Verwendung einer Verbindung der Formel I gemäß Anspruch 1 und/oder eines ihrer physiologisch unbedenklichen Salze zur Herstellung von Arzneimitteln.

9. Verwendung einer Verbindung der Formel I gemäß Anspruch 1 und/oder eines ihrer physiologisch unbedenklichen Salze bei der Bekämpfung von Krankheiten.
